# EUROPEAN PATENT APPLICATION

(11) **EP 3 769 730 A1**
(43) Date of publication of application: **27.01.2021**
(21) Application number: 20187593.7
(22) Date of filing: 24.07.2020
(51) Int. Cl.: A61F 13/00, A61L 15/60, B23K 26/00

(54) **LASER PROCESSING OF FOAM FOR IMPROVED PERFORMANCE IN NEGATIVE PRESSURE WOUND THERAPY**

(30) Priority: 24.07.2019 US 201962878204 P
(71) Applicant: Preco, Inc., Somerset, WI 54025 (US)
(72) Inventor: HILL, Kimberly, South St. Paul, Minnesota 55075 (US); ARVEY, Ken, Emerald, Wisconsin 54013 (US)
(74) Representative: Downing, Michael Philip

(57) **Abstract**

A negative pressure wound dressing and method of making same includes a flexible foamed polymeric material having a plurality of channels or holes formed by a laser within the cellular structure thereof, the flexible foamed polymeric material for use to contact a wound contact surface in negative pressure wound treatment.

## Description

### CROSS-REFERENCE TO RELATED APPLICATION

The present application claims priority to and the benefit of U.S. provisional application Serial No. 62/878,204, filed on July 24, 2019, the contents of which are hereby incorporated in their entirety.

### BACKGROUND

This invention relates to a negative pressure wound dressing system and a method of making such a wound dressing for use in negative pressure wound treatment.

The body's natural wound healing process is a complex series of events beginning at the moment of injury. Initially the body reacts by delivering proteins and other factors to the wound through the blood stream to minimize the damage. Blood clots to prevent blood loss while cells engulf bacteria and debris to carry it away from the wound site. Next, the body begins to repair itself in a stage of healing often referred to as the proliferate phase. This phase is characterized by the deposition of granulation tissue in the wound bed. Granulation tissue provides a base structure over which cells may migrate inwardly from the periphery to close the wound. Finally, the process ends as collagen gives strength to new tissue over time often forming a scar.

One technique for promoting the natural healing process, particularly, but not exclusively during the proliferate phase, is known as negative pressure wound therapy (NPWT). Negative pressure wound therapy (NPWT) has gained in popularity after Morykwas and Argenta et al. published seminal papers on the Vacuum-Assisted Closure device (V.A.C.®) in 1997. Its use has changed the way in which many wounds are treated. The relevant dressings include a suitable open cell foam, placed in or on the wound, and covered by an occlusive adherent film dressing. Tubing, placed in contact with the foam, beneath and sealed off by the adherent film, is attached to a source of suction that causes the creation of a negative pressure within the open cell foam.

Application of reduced pressure over a wound has been found to assist in closing the wound. The reduced pressure may be effective to promote blood flow to the area, to stimulate the formation of granulation tissue, and the migration of healthy tissue over the wound by the natural process. Also reduced pressure may inhibit bacterial growth by assisting in removing fluids exuding from the wound. This technique not only has proven effective for chronic or non-healing wounds but has also been used for other purposes such as post-operative wound care.

Although NPWT dressings work well, the open cell foam nature of the NPWT dressing results in the wound contact surface becoming occluded with solids from the exudate being drawn into the cellular structure from the negative pressure

### SUMMARY

The present disclosure describes a negative pressure wound dressing that has a plurality of channels or holes formed by a laser within the cellular structure of a flexible foamed polymeric material for use at the wound contact surface in negative pressure wound treatment (NPWT).

In another aspect of the negative pressure wound dressing of this disclosure, in one or more embodiments, a portion of the flexible foamed polymeric material comprises a wound contact surface having a topography comprising channels or holes within the flexible foamed polymeric material.

In another aspect of the negative pressure wound therapy dressing of this disclosure, in one or more embodiments, the flexible foamed polymeric material has a portion of the topography processed by a laser and is characterized by minimal reduction in fluid flow in areas that were processed by the laser.

This disclosure also describes a method which includes laser processing a flexible foamed polymeric material whereby a portion of the foamed material is removed by the laser without affecting the cellular structure or reducing the fluid flow characteristics of the flexible foamed polymeric material. As used throughout the disclosure, what is meant by the term "fluid" is a gas or liquid, or a combination of both.

This disclosure also describes wherein in one or more embodiments when a portion of the foamed material is removed at least one channel is produced, and the at least one channel is configured to allow or enhance the flow of fluids from the wound and/or to an attached negative pressure device.

This disclosure also describes wherein in one or more embodiments when a portion of the foamed material that is removed at least one channel is produced, and the at least one channel is configured to allow or enhance movement of water and/or gases through the foam and out of the wound.

This disclosure also describes wherein in one or more embodiments when a portion of the foamed material is removed at least one channel is produced, and the at least one channel is configured to allow or enhance the movement of water and/or gases through the foam and into the wound.

This disclosure also describes wherein in one or more embodiments when a portion of the foamed material is removed at least one channel and/or openings are produced, and the at least one channel and/or openings are configured to enable the addition of medications, growth stimulators, scaffolding material, or biomaterial, cells, and/or tissue to the wound area.

This disclosure also describes wherein in one or more embodiments when a portion of the foamed material is removed and at least one channel is produced, and the at least one channel is configured to allow liquid ingress and/or allow the wound to be flushed in conjunction with an attached negative pressure device.

In another aspect of this disclosure in one or more embodiments the flexible foam polymeric material is subsequently used in negative pressure wound treatment.

In another aspect of this disclosure, in one or more embodiments, the portion of the flexible foamed polymeric material that is removed results in a change in topography of a wound contact surface of the flexible foamed polymeric material, or production of channels or holes within the flexible foamed polymeric material.

In another aspect of this disclosure, in one or more embodiments, the portion of the foamed material being removed is digitally controlled.

In another aspect of this disclosure, in one or more embodiments, the flexible foamed polymeric material produced by the process of this disclosure is characterized by minimal reduction in fluid flow in areas that were processed by the laser.

This disclosure also describes, in one or more embodiments, a flexible foamed polymeric material having a surface topography or internal structure processed by a laser wherein portions of the flexible foamed polymeric material are removed due to laser processing.

This disclosure also describes, in one or more embodiments, a foam configured by a laser to be attached to and/or removed from solid or semisolid scaffolding introduced to the wound to support tissue or bone growth.

This disclosure further describes the portion of the foamed material being removed by the laser beam is configured to deliver fluids to the wound or wound area.

This disclosure also describes the portion of the foamed material being removed by the laser beam is configured to transport fluids to or from the wound or wound area

### BRIEF DESCRIPTION OF THE DRAWINGS

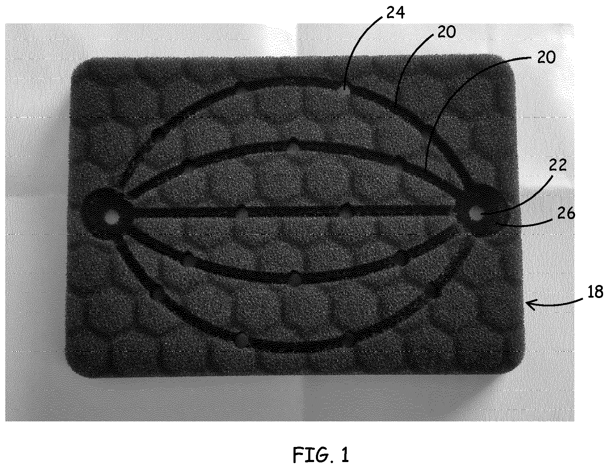

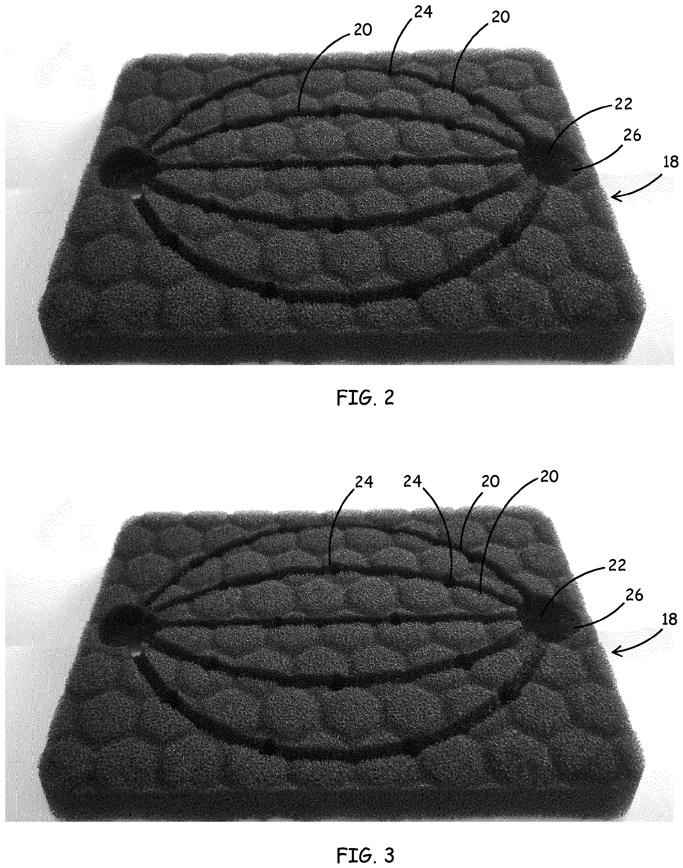

FIG. 1-3 are photographic views of a flexible foamed polymeric material of this disclosure with through holes and channels created by laser processing.

### DETAILED DESCRIPTION

In general, dressings for negative pressure wound therapy comprise an open cellular foam material comprising a wound contact surface that is placed in contact with the wound. It is to be understood that the term "negative pressure" as used in this specification is intended to mean reduced atmospheric pressure that is negative relative to atmospheric pressure in the sense in which that term is commonly used in the art.

On a side opposing the contact surface, a cover layer encompasses the NPWT dressing and establishes a barrier such that a negative pressure may be maintained within the NPWT dressing. There may be other layers between the cellular foam material and the cover layer; however, the other layers are such that they do not affect the maintenance of the negative pressure within the dressing and at the wound contact surface of the cellular foam material.

This disclosure describes the use of a laser to produce channels and through holes to enhance the performance of foam materials to collect and transport exudate from the wound during negative pressure wound therapy (NPWT). In addition, channels can be produced that deliver gasses or liquids or combinations thereof to the wound or wound area. Either way, the laser produced channels are formed without compression of the cellular structure that defines the channel. Thus the fluid flow characteristics of the channel have not been impaired by the formation of the channel and are similar when compared to a channel formed by conventional methods in a non-compressible material. By fluid flow characteristics it is meant the ability of the channel to handle either exudates or fluids. As described herein, laser modified foams have the ability to increase performance of NPWT dressings in the following ways:
1. Laser processed foam materials enhance the exudate handling capability when used to create capillary openings in the foam material. Such capillary openings can transport the exudate from the wound area to an exudate holding mechanism such as a vacuum collection chamber. In addition, laser produced holes, channels or pockets in the foam can increase the exudate holding capacity of the foam as compared to foamed materials not having been processed in this manner.
2. Laser processed foam materials can prolong the effectiveness of NPWT wound care devices in both time duration and efficacy. By creating channels and pathways within the foam materials the foam material is able to transport larger amounts of exudate and are significantly less likely to become obstructed, thus the effectiveness of the wound care device is increased and by increasing the flow rate and reducing blockage in wound care foam, the duration of time that the dressing is on the wound is extended.
3. Laser processed foams used in NPWT have the added advantage of being shaped into configurations not possible using traditional methods such as flatbed or rotary die presses. Due to the non-contact nature of digital laser processing the fluid transfer nature of the material can be enhanced and shaped to permit custom designs that reduce the required vacuum to be efficacious. Lower levels of vacuum can reduce the size and complexity of NPWT devices.

As illustrated in FIGS. 1-3, laser processing produces well defined channels 20 and though holes 24 and 22 in the flexible foamed polymeric material 18. Laser processing can also produce a well configuration 26 surrounding through hole 22. Virtually any configuration can be produced in or on the flexible foamed polymeric material through the use of a laser.

The type of open cellular foam that is suitable for an NPWT dressing within this disclosure is a flexible open cell foamed material that adapts to the contour of the body where the wound exists. Flexibility of the dressing is important not only for contact with the wound but also to avoid unnecessary "hard" contact with the wound which in turn may cause discomfort and even pain to the wounded person.

For purposes of this disclosure the term "foam" shall mean a solid phase containing a dispersed gas phase. The gas phase is the result of either incorporating the gas through high shear mixing into the foam or introducing a blowing agent that reacts to produce gas. Suitable foamed materials are generally referred to as reticulated foams. Suitable reticulated foams for purposes of this disclosure include polymeric foams such as foamed polyurethanes. Reticulated foams are very porous, low density, solid foams. Generally, reticulated foams have a very open cell structure. By open cell structure for purposes of this disclosure is meant that there are few walls between cells such that fluid or air readily flows through the cellular structure. Suitable foams for NPWT dressings are well known in the art.

The preferred flexible open cell structure of the foamed material presents a problem in subsequent processing of the foamed material. Any type of physical contact with the open cell foamed material results in the material compressing or giving way to the contact. For example, to cut the foamed material with a cutting blade will result in the foamed material being squeezed initially before any cut is made thereby affecting the accuracy of the cut. Making holes in the foamed material using a mechanical hole cutter results in the foamed material being cut through all the way since the foamed material will be compressed before any cutting action results. Precisely cutting contours the flexible foamed polymeric material is virtually impossible using a cutting blade.

It has been found that the use of a laser to engage the surface of the foamed material results in the ability to cut precise channels, contoured surfaces within the contact surface of the NPWT dressing, and even partial holes which may extend a selected distance within the foamed material. When the foamed material is laser processed, there is no physical contact and therefore no compression of the foamed surface thereby permitting changes in the topography of the foamed surface that contacts the wound. Such changes to the topography of a flexible foam were not possible utilizing prior art methods.

If the foamed material is compressed prior to laser processing, the partial holes made during a compressed state upon decompression of the foamed material will result in certain sections becoming reservoirs or wells wider than the laser focal point used to make the initially compressed partial hole.

One advantage that has been found with changing the topography of the contact surface of the foamed material utilizing a laser is that additional surface area is created at the point of contact with the wound. Such additional surface area may extend the effective life of the NPWT dressing and/or increase its effectiveness in processing exudate from the wound.

Another advantage of laser processing when compared to physical processing such as with a cutting blade of the foamed material is that the compression used is of such force that the open cell structure of the section being cut may result in cellular walls being fused and thereby affecting the flow characteristics of the foamed material. This would be a great disadvantage for use in an NPWT dressing. Consequently through holes made with a laser of this disclosure are physically different than through holes made with a cutting blade.

Another advantage of laser processing is that the selected pattern, e.g. through holes, partial holes, channels, topographical changes to the surface of the foam material may be mapped and repeated digitally. Digital control provides for easier customization of the dressing to address different wound situations.

Suitable polymers that are within this disclosure may be thermoplastics or thermoset polymers. The criteria for this disclosure are that the polymer when foamed results in a flexible open cell structure as defined herein. Polymers commonly used for NPWT dressings include but are not limited to polyurethanes, polyethers and polyesters.

The foam may also be configured by a laser for attachment to and/or removal from solid or semisolid scaffolding introduced to the wound to support tissue or bone growth. Scaffold as the term is used herein means a framework that serves as a platform for cellular localization, adhesion and differentiation as well as serving as a guide for development of new functional tissue. Most often scaffolds are used for skin regeneration and to prevent scarring. Such scaffolds are well known.

Although the present disclosure has been described with reference to preferred embodiments, workers skilled in the art will recognize that changes may be made in form and detail without departing from the spirit and scope of the disclosure.

## Claims

1. A negative pressure wound dressing comprising a flexible foamed polymeric material having a plurality of channels or holes formed by a laser, the holes formed within the cellular structure thereof, the flexible foamed polymeric material for use to contact a wound in negative pressure wound treatment.

2. The negative pressure wound dressing of claim 1 wherein a portion of the flexible foamed polymeric material comprises a wound contact surface having a topography comprising channels or holes within the flexible foamed polymeric material.

3. The negative pressure wound dressing of claim 1 or 2 wherein a portion of the topography of the flexible foamed polymeric material was processed by a laser and is **characterized by** minimal reduction in fluid flow in areas that were processed by the laser.

4. A method comprising laser processing a flexible foamed polymeric material whereby a portion of the foamed material is removed by a laser beam without otherwise affecting the cellular structure of the material or reducing the fluid flow characteristics of the flexible foamed polymeric material.

5. The method of claim 4 wherein the portion of the flexible foamed polymeric material that is removed results in a change in topography of a wound contact surface of the flexible foamed polymeric material or production of channels or holes within the flexible foamed polymeric material.

6. The method of claim 4 or 5 wherein the portion of the foamed material being removed is digitally controlled.

7. The method of claim 4 wherein the flexible foamed polymeric material produced by the process of this disclosure is **characterized by** minimal reduction in fluid flow in areas that were processed by the laser.

8. The method of any one of claims 4-7 wherein the portion of the foamed material that is removed produces at least one channel, and the at least one channel is configured to allow or enhance the flow of fluids from the wound and/or to an attached negative pressure device.

9. The method of any one of claims 4-7 wherein the portion of the foamed material that is removed produces at least one channel, and the at least one channel is configured to allow or enhance the movement of water and/or gases through the foam and out of the wound.

10. The method of any one of claims 4-9 wherein the portion of the foamed material that is removed produces at least one channel and/or openings, and the at least one channel and/or openings are configured to enable the addition of medications, growth stimulators, scaffolding material, or biomaterial, cells, and/or tissue to the wound area.

11. The method of any one of claims 4 to 10 wherein the flexible foam polymeric material is subsequently used in negative pressure wound treatment.

12. The method of any one of the preceding claims wherein the portion of the foamed material that is removed produces at least one channel, and the at least one channel is configured to allow liquid ingress and/or allow the wound to be flushed in conjunction with an attached negative pressure device.

13. The method of any one of claims 4 to 12 whereby the portion of the foamed material removed by the laser beam is configured to deliver fluids to the wound or wound area or to transport fluids to or from the wound or wound area.

14. A foam configured by a laser to be attached to and/or removed from solid or semisolid scaffolding introduced to the wound to support tissue or bone growth.
